(19)

**Europäisches Patentamt**
**European Patent Office**
**Office européen des brevets**

(11) **EP 4 636 772 A1**

(12) **EUROPEAN PATENT APPLICATION**
published in accordance with Art. 153(4) EPC

(43) Date of publication:
**22.10.2025 Bulletin 2025/43**

(21) Application number: **22968092.1**

(22) Date of filing: **12.12.2022**

(51) International Patent Classification (IPC):
**G16B 30/00** $^{(2019.01)}$     **C12Q 1/68** $^{(2018.01)}$

(52) Cooperative Patent Classification (CPC):
**C12Q 1/68; G16B 30/00**

(86) International application number:
**PCT/CN2022/138467**

(87) International publication number:
**WO 2024/124378 (20.06.2024 Gazette 2024/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **MGI Tech Co., Ltd.**
**Shenzhen, Guangdong 518083 (CN)**

(72) Inventors:
• **DING, Ya**
  **Shenzhen, Guangdong 518083 (CN)**
• **LIU, Liyi**
  **Shenzhen, Guangdong 518083 (CN)**
• **ZHANG, Liangliang**
  **Shenzhen, Guangdong 518083 (CN)**

• **XU, Dongyang**
  **Shenzhen, Guangdong 518083 (CN)**
• **LUO, Bin**
  **Shenzhen, Guangdong 518083 (CN)**
• **WANG, Le**
  **Shenzhen, Guangdong 518083 (CN)**
• **ZHANG, Yuannian**
  **Shenzhen, Guangdong 518083 (CN)**
• **OUYANG, Kai**
  **Shenzhen, Guangdong 518083 (CN)**
• **ZHANG, Yinghua**
  **Shenzhen, Guangdong 518083 (CN)**
• **GONG, Meihua**
  **Shenzhen, Guangdong 518083 (CN)**
• **XU, Chongjun**
  **Shenzhen, Guangdong 518083 (CN)**

(74) Representative: **Dehns**
**10 Old Bailey**
**London EC4M 7NG (GB)**

(54) **METHOD FOR CORRECTING BASE INTERPRETATION RESULT OF SYNCHRONOUS SEQUENCING, SYNCHRONOUS SEQUENCING METHOD AND SYSTEM, AND COMPUTER PROGRAM PRODUCT**

(57)     Provided is a synchronous sequencing method, including: constructing a sequencing library for a nucleic acid sample to be tested; loading the sequencing library onto a sequencing chip; performing a plurality of synchronous sequencing reaction cycles on the sequencing library, wherein an image set generated in each of the plurality of synchronous sequencing reaction cycles constitutes a raw image set of the synchronous sequencing; acquiring a base-calling result of the synchronous sequencing based on the raw image set of the synchronous sequencing; correcting the signal intensity value of each base channel based on a predetermined correction parameter to obtain a corrected base-calling result; and determining a base output result of the synchronous sequencing based on the corrected base-calling result.

EP 4 636 772 A1

## Description

### FIELD

The present disclosure relates to the field of biological information. Specifically, the present disclosure relates to a method for correcting a base-calling result of synchronous sequencing and a synchronous sequencing method.

### BACKGROUND

The base recognition algorithm for high-throughput sequencing is called the base-calling algorithm. Commonly used software for base-calling algorithm includes Zebracall and Litecall software that are compatible with sequencers from MGI®, as well as Bustard software from Illumina®. Based on the current sequencing principle (one base per sequencing unit), existing base-calling software is designed to recognize a single base.

Synchronous sequencing refers to a sequencing method in which first- and second-strand templates are generated simultaneously, and the signal intensities of the first strand and the second strand are regulated by controlling the amplification time of the first strand and the second strand. The use of synchronous sequencing can improve sequencing throughput and reduce sequencing costs.

Existing base-calling software, such as Bustard or Ibis, is only designed for recognizing a single base, meaning that in each cycle of biochemical reaction, only one base emits fluorescence per fluorescent unit. However, for signal data obtained from "synchronous sequencing" involving simultaneously sequencing from both ends of DNA, current algorithms can recognize only one of the two actual bases or none in some cases, thereby failing to meet the base-calling requirements of synchronous sequencing.

Therefore, there is an urgent need for a new algorithm capable of performing base recognition for synchronous sequencing.

### SUMMARY

The present disclosure is intended to solve, at least to some extent, the technical problems existing in the prior art. To this end, the present disclosure provides a synchronous sequencing method, a method for correcting a base-calling result of synchronous sequencing, a synchronous sequencing system, and a computer program product. The use of the method and system of the present disclosure can accurately recognize base combinations of synchronous sequencing, greatly reduce sequencing time and costs, improve the sequencing throughput, and are suitable for widespread application.

In an aspect of the present disclosure, a synchronous sequencing method is provided. According to an embodiment of the present disclosure, the method includes: constructing a sequencing library for a nucleic acid sample to be tested; loading the sequencing library onto a sequencing chip, wherein the sequencing chip is provided with at least one composite template sample spot, the composite template sample spot being provided with at least one sequencing template; performing a plurality of synchronous sequencing reaction cycles on the sequencing library, wherein an image set generated in each of the plurality of synchronous sequencing reaction cycles constitutes a raw image set of the synchronous sequencing; acquiring a base-calling result of the synchronous sequencing based on the raw image set of the synchronous sequencing, wherein the base-calling result includes a signal intensity value of each base channel in each of the plurality of synchronous sequencing reaction cycles; correcting the signal intensity value of each base channel based on a predetermined correction parameter to obtain a corrected base-calling result, wherein the correction parameter includes at least one of a crosstalk correction parameter and a phasing correction parameter for each base channel; and determining a base output result of the synchronous sequencing based on the corrected base-calling result.

According to an embodiment of the present disclosure, the inventors have discovered that crosstalk will occur among the four channels during the sequencing process, such as optical signal crosstalk between base A channel and base T channel, or optical signal crosstalk between base C channel and base G channel, resulting in inaccurate detection results of each base channel. This deviation on synchronous sequencing can be particularly significant, which results in an inaccurate distinction between two bases, thereby possibly rendering the sequencing results unusable. As a result, it is necessary to perform crosstalk correction on the signal intensities of each base channel in the image obtained from the sequencing reaction.

In addition, according to an embodiment of the present disclosure, the inventors have also found that the previous or subsequent sequencing cycle will cause lagging or leading signal interference for the current sequencing cycle. As a result, it is also necessary to perform phasing correction on the sequencing signals obtained from the current sequencing cycle using the sequencing signals from the previous or subsequent sequencing cycle. Thus, correcting the signal data of each base channel achieves the purpose of improving data authenticity and eliminating noise and can obtain the calling information of the corrected base combination in synchronous sequencing.

According to an embodiment of the present disclosure, the above synchronous sequencing method may further include the following additional technical features.

According to an embodiment of the present disclosure, the sequencing templates are located at different positions on the same nucleic acid molecule.

According to an embodiment of the present disclosure, the sequencing templates are located on different nucleic acid molecules.

According to an embodiment of the present disclosure, the sequencing templates are located at different positions on the same DNA nanoball.

According to an embodiment of the present disclosure, the sequencing templates are located on different strands of the same DNA nanoball.

According to an embodiment of the present disclosure, signal quantities generated by the plurality of sequencing templates follow a predetermined relationship.

According to an embodiment of the present disclosure, the correction parameter is determined by: identifying a plurality of high-confidence composite sample spots among the plurality of composite template sample spots based on the base-calling result; identifying, for a given base channel, a plurality of first reference sample spots and a plurality of second reference sample spots among the plurality of high-confidence composite sample spots, wherein the plurality of first reference sample spots are selected from crosstalk correction parameter reference sample spots and the plurality of second reference sample spots are selected from phasing correction parameter reference sample spots; and identifying, for the given base channel, the crosstalk correction parameter of the given base channel based on a base-calling result of the plurality of first reference sample spots and the phasing correction parameter of the given base channel based on a base-calling result of the plurality of second reference sample spots.

According to an embodiment of the present disclosure, the plurality of high-confidence composite sample spots includes a composite sample spot where the base-calling result indicates only one type of base in a given sequencing reaction cycle.

According to an embodiment of the present disclosure, for the given base channel, the plurality of first reference sample spots include a composite sample spot satisfying the following condition: in the given sequencing reaction cycle, the base-calling result of the composite sample spot indicates only one type of base that is different from the given base.

According to an embodiment of the present disclosure, the crosstalk correction parameter is obtained by training the following formula with a signal intensity value of each base channel from the plurality of first reference sample spots:

$$yi_{(B1,N)} = \beta 0 + \beta 1 * Xi_{(B2,N)} + \beta 2 * Xi_{(B3,N)} + \beta 3 * Xi_{(B4,N)} + \in$$

where: B1, B2, B3, and B4 represent one of base A channel, base T channel, base G channel, and base C channel, respectively, with B1 representing the given base channel; N represents the serial number of the given cycle; $yi_{(B1,N)}$ represents the signal intensity value of the given base channel in the given cycle N; $Xi_{(B2,N)}$, $Xi_{(B3,N)}$, and $Xi_{(B4,N)}$ represent signal intensity values of given base channels B2, B3, and B4 in the given cycle N, respectively; $\beta 0$, $\beta 1$, $\beta 2$, and $\beta 3$ represent crosstalk correction parameters for the given base channel; and $\in$ represents an error parameter. The formula is trained with a regression model.

According to an embodiment of the present disclosure, for the given base channel, the plurality of second reference sample spots are composite sample spots that satisfy the following conditions: (A) in the given sequencing reaction cycle, the base-calling result of the composite sample spot indicates only one type of base that is different from the given base; and (B) in at least one of the previous or subsequent cycles of the given sequencing reaction cycle, the base-calling result of the composite sample spot indicates only one type of the given base.

According to an embodiment of the present disclosure, in condition (B), if in the previous cycle of the given sequencing reaction cycle, the base-calling result of the composite sample spot indicates only one type of the given base, the second reference sample spot is identified as a third reference sample spot, wherein the third reference sample spot is selected from lagging phasing correction parameters reference sample spot; or in condition (B), if in the subsequent cycle of the given sequencing reaction cycle, the base-calling result of the composite sample spot indicates only one type of the given base, the second reference sample spot is identified as a fourth reference sample spot, wherein the fourth reference sample spot is selected from leading phasing correction parameters reference sample spot.

According to an embodiment of the present disclosure, the phasing correction parameter further includes at least one of lagging phasing correction parameters and leading phasing correction parameters, and the phasing correction parameter is obtained by training the following formula with a signal intensity value of each base channel from the plurality of second reference sample spots:

$$yi_{(B1,M)} = \beta01 + B4 * Xi_{(B1,M-1)}$$

or,

$$yi_{(B1,M)} = \beta02 + B5 * Xi_{(B1,M+1)}$$

where: B1 represents the given base channel, M represents the serial number of the given cycle, M+1 represents the number of the subsequent cycle of the given cycle, M-1 represents the number of the previous cycle of the given cycle; $\beta01$ and B4 represent lagging phasing correction parameters for the given base channel, and $\beta02$ and B5 represent leading phasing correction parameters for the given base channel. The formula is trained with a regression model.

According to an embodiment of the present disclosure, the method further includes inputting the corrected base-calling result as an input feature into a machine learning model to output a base combination of synchronous sequencing. The machine learning model is trained in a supervised manner using a reference sequence with a predetermined sequence as a training set. The reference sequence is subjected to synchronous sequencing, and a base-calling result obtained from a raw image of synchronous sequencing is corrected to generate a corrected base-calling result of each cycle as an input feature. The base combination in the reference sequence corresponding to the base-calling result of each cycle is used as a label. The machine learning model is at least one of Bayesian, SVM, KNN, Random Forest, XGBoost, and Neural Network.

In yet another aspect of the present disclosure, a method for correcting a base-calling result of synchronous sequencing is provided. According to an embodiment of the present disclosure, the method includes: acquiring a raw image set of the synchronous sequencing, wherein in the synchronous sequencing, at least one composite template sample spot is provided, at least one sequencing template is provided in the composite template sample spot, with a plurality of sequencing reaction cycles being performed on the at least one sequencing template, and an image set generated in each of the plurality of sequencing reaction cycles constitutes the raw image set; acquiring a base-calling result of the synchronous sequencing based on the raw image set, wherein the base-calling result includes a signal intensity value of each base channel in each of the plurality of sequencing reaction cycles; and correcting the signal intensity value of each base channel based on a predetermined correction parameter to obtain a corrected base-calling result, wherein the correction parameter includes at least one of a crosstalk correction parameter and a phasing correction parameter for each base channel.

According to an embodiment of the present disclosure, the correction parameter is determined by: identifying a plurality of high-confidence composite sample spots among the at least one composite template sample spot based on the base-calling result; identifying, for a given base channel, a plurality of first reference sample spots and a plurality of second reference sample spots among the plurality of high-confidence composite sample spots, wherein the plurality of first reference sample spots are selected from crosstalk correction parameter reference sample spots and the plurality of second reference sample spots are selected from phasing correction parameter reference sample spots; and identifying, for the given base channel, the crosstalk correction parameter of the given base channel based on a base-calling result of the plurality of first reference sample spots and the phasing correction parameter of the given base channel based on a base-calling result of the plurality of second reference sample spots, wherein the plurality of high-confidence composite sample spots includes a composite sample spot where the base-calling result indicates only one type of base in a given sequencing reaction cycle.

According to an embodiment of the present disclosure, for the given base channel, the plurality of first reference sample spots include a composite sample spot satisfying the following condition: in the given sequencing reaction cycle, the base-calling result of the composite sample spot indicates only one type of base that is different from the given base; for the given base channel, the plurality of second reference sample spots are composite sample spots that satisfy the following conditions: (A) in the given sequencing reaction cycle, the base-calling result of the composite sample spot indicates only one type of base that is different from the given base; and (B) in at least one of the previous or subsequent cycles of the given sequencing reaction cycle, the base-calling result of the composite sample spot indicates only one type of the given base; wherein in condition (B), if in the previous cycle of the given sequencing reaction cycle, the base-calling result of the composite sample spot indicates only one type of the given base, the second reference sample spot is identified as a third reference sample spot, wherein the third reference sample spot is selected from lagging phasing correction parameters reference sample spot; or in condition (B), if in the subsequent cycle of the given sequencing reaction cycle, the base-calling result of the composite sample spot indicates only one type of the given base, the second reference sample spot is identified as a fourth reference sample spot, wherein the fourth reference sample spot is selected from leading phasing correction parameters reference sample spot.

According to an embodiment of the present disclosure, the correction parameter is determined by: identifying a plurality of high-confidence composite sample spots among the at least one composite template sample spot based on the base-calling result; identifying, for a given base channel, a plurality of first reference sample spots and a plurality of second reference sample spots among the plurality of high-confidence composite sample spots, wherein the plurality of first reference sample spots are selected from crosstalk correction parameter reference sample spots and the plurality of second reference sample spots are selected from phasing correction parameter reference sample spots; and identifying, for the given base channel, the crosstalk correction parameter of the given base channel based on a base-calling result of the plurality of first reference sample spots and the phasing correction parameter of the given base channel based on a base-calling result of the plurality of second reference sample spots, wherein the plurality of high-confidence composite sample spots includes a composite sample spot where the base-calling result indicates only one type of base in a given sequencing reaction cycle.

According to an embodiment of the present disclosure, for the given base channel, the plurality of first reference sample spots include a composite sample spot satisfying the following condition: in the given sequencing reaction cycle, the base-calling result of the composite sample spot indicates only one type of base that is different from the given base; for the given base channel, the plurality of second reference sample spots are composite sample spots that satisfy the following conditions: (A) in the given sequencing reaction cycle, the base-calling result of the composite sample spot indicates only one type of base that is different from the given base; and (B) in at least one of the previous or subsequent cycles of the given sequencing reaction cycle, the base-calling result of the composite sample spot indicates only one type of the given base; wherein in condition (B), if in the previous cycle of the given sequencing reaction cycle, the base-calling result of the composite sample spot indicates only one type of the given base, the second reference sample spot is identified as a third reference sample spot, wherein the third reference sample spot is selected from lagging phasing correction parameters reference sample spot; or in condition (B), if in the subsequent cycle of the given sequencing reaction cycle, the base-calling result of the composite sample spot indicates only one type of the given base, the second reference sample spot is identified as a fourth reference sample spot, wherein the fourth reference sample spot is selected from leading phasing correction parameters reference sample spot.

According to an embodiment of the present disclosure, the crosstalk correction parameter is obtained by training the following formula with a signal intensity value of each base channel from the plurality of first reference sample spots:

$$yi_{(B1,N)} = \beta 0 + \beta 1 * Xi_{(B2,N)} + \beta 2 * Xi_{(B3,N)} + \beta 3 * Xi_{(B4,N)} + \in$$

where: B1, B2, B3, and B4 represent one of base A channel, base T channel, base G channel, and base C channel, respectively, with B1 representing the given base channel; N represents the serial number of the given cycle; $yi_{(B1,N)}$ represents the signal intensity value of the given base channel in the given cycle N; $Xi_{(B2,N)}$, $Xi_{(B3,N)}$, and $Xi_{(B4,N)}$ represent signal intensity values of given base channels B2, B3, and B4 in the given cycle N, respectively; $\beta 0$, $\beta 1$, $\beta 2$, and $\beta 3$ represent crosstalk correction parameters for the given base channel; and $\in$ represents an error parameter. The phasing correction parameter further includes at least one of lagging phasing correction parameters and leading phasing correction parameters, and the phasing correction parameter is obtained by training the following formula with a signal intensity value of each base channel from the plurality of second reference sample spots:

$$yi_{(B1,M)} = \beta 01 + B4 * Xi_{(B1,M-1)}$$

or,

$$yi_{(B1,M)} = \beta 02 + B5 * Xi_{(B1,M+1)}$$

where: B1 represents the given base channel, M represents the serial number of the given cycle, M+1 represents the number of the subsequent cycle of the given cycle, M-1 represents the number of the previous cycle of the given cycle, $\beta 01$ and B4 represent lagging phasing correction parameters for the given base channel, and $\beta 02$ and B5 represent leading phasing correction parameters for the given base channel. The formula is trained with a regression model.

In yet another aspect of the present disclosure, a system of synchronous sequencing is provided. According to an embodiment of the present disclosure, the system includes: a sequencing chip, provided with at least one composite template sample spot, the composite template sample spot being provided with at least one sequencing template; a detection device, configured to perform a plurality of synchronous sequencing reaction cycles on a sequencing library, wherein an image set generated in each of the plurality of synchronous sequencing reaction cycles constitutes a raw image set of the synchronous sequencing; and one or more processors, configured to execute: (A) acquiring a base-calling result of the synchronous sequencing based on the raw image set of the synchronous sequencing, wherein the base-calling result includes a signal intensity value of each base channel in each of the plurality of synchronous sequencing reaction cycles, (B) correcting the signal intensity value of each base channel based on a predetermined correction parameter to obtain a corrected base-calling result, wherein the correction parameter includes at least one of a crosstalk correction parameter and a phasing correction parameter for each base channel, and (C) determining a base output result of the synchronous sequencing based on the corrected base-calling result.

According to an embodiment of the present disclosure, the processor is further configured to execute a crosstalk correction parameter acquisition module, the crosstalk correction parameter acquisition module being configured to obtain the crosstalk correction parameter by training the following formula with the signal intensity value of each base channel from the plurality of first reference sample spots:

$$yi_{(B1,N)} = \beta 0 + \beta 1 * Xi_{(B2,N)} + \beta 2 * Xi_{(B3,N)} + \beta 3 * Xi_{(B4,N)} + \in$$

where: B1, B2, B3, and B4 represent one of base A channel, base T channel, base G channel, and base C channel, respectively, with B1 representing the given base channel; N represents the serial number of the given cycle; $yi_{(B1,N)}$ represents the signal intensity value of the given base channel in the given cycle N; $Xi_{(B2,N)}$, $Xi_{(B3,N)}$, and $Xi_{(B4,N)}$ represent signal intensity values of given base channels B2, B3, and B4 in the given cycle N, respectively; $\beta 0$, $\beta 1$, $\beta 2$, and $\beta 3$ represent crosstalk correction parameters for the given base channel; and $\in$ represents an error parameter; and the plurality of first reference sample spots are selected from crosstalk correction parameter reference sample spots, and the plurality of first reference sample spots include a composite sample spot satisfying the following condition: in the given sequencing reaction cycle, the base-calling result of the composite sample spot indicates only one type of base that is different from the given base.

According to an embodiment of the present disclosure, the processor is further configured to execute a phasing correction parameter acquisition module, the phasing correction parameter acquisition module being configured to obtain the phasing correction parameter by training the following formula with the signal intensity value of each base channel from the plurality of second reference sample spots:

$$yi_{(B1,M)} = \beta 01 + B4 * Xi_{(B1,M-1)}$$

or,

$$yi_{(B1,M)} = \beta 02 + B5 * Xi_{(B1,M+1)}$$

where: B1 represents the given base channel, M represents the serial number of the given cycle, M+1 represents the number of the subsequent cycle of the given cycle, M-1 represents the number of the previous cycle of the given cycle, $\beta 01$ and B4 represent lagging phasing correction parameters for the given base channel, and $\beta 02$ and B5 represent leading phasing correction parameters for the given base channel; the plurality of second reference sample spots are composite sample spots that satisfy the following conditions: (A) in the given sequencing reaction cycle, the base-calling result of the composite sample spot indicates only one type of base that is different from the given base; and (B) in at least one of the previous or subsequent cycles of the given sequencing reaction cycle, the base-calling result of the composite sample spot indicates only one type of the given base; and the crosstalk correction parameter acquisition module or phasing correction parameter acquisition module includes a regression model that is used for training the formula.

In yet another aspect of the present disclosure, an electronic device is provided. According to an embodiment of the present disclosure, the electronic device includes a memory and a processor, wherein the memory has a program stored thereon that is executable by the processor, and the program, when executed by the processor, implements the method for correcting a base-calling result of synchronous sequencing.

In yet another aspect of the present disclosure, a computer-readable storage medium is provided. According to an embodiment of the present disclosure, the computer-readable storage medium has one or more programs stored thereon that are executable by one or more processors to implement the method for correcting a base-calling result of synchronous sequencing.

Additional aspects and advantages of the present disclosure will be partially set forth in the following description below and will be partially apparent from the description.

## BRIEF DESCRIPTION OF THE DRAWINGS

The above and/or additional aspects and advantages of the present disclosure will become apparent and readily appreciated from the following description of the embodiments, taken in conjunction with the accompanying drawings, in which:

FIG. 1 shows a schematic flow diagram of a synchronous sequencing method according to an embodiment of the present disclosure;
FIG. 2 shows scatter plots of initial signals between every two of four channels under synchronous sequencing conditions according to an embodiment of the present disclosure;
FIG. 3 shows scatter plots of initial signals between every two of four channels under synchronous sequencing conditions according to another embodiment of the present disclosure;
FIG. 4 shows a 3D signal distribution diagram in which the sixteen base combinations of signals in synchronous sequencing are classified into four categories based on the maximum signal according to an embodiment of the present disclosure;
FIG. 5 shows a flow diagram for constructing a machine learning model according to an embodiment of the present disclosure;
FIG. 6 shows a flow diagram for determining a base-calling result of synchronous sequencing according to an embodiment of the present disclosure;
FIG. 7 shows a schematic structural diagram of a synchronous sequencing system according to an embodiment of the present disclosure; and
FIG. 8 shows a schematic diagram of a memory according to an embodiment of the present disclosure.

## DETAILED DESCRIPTION

The embodiments of the present disclosure will be described in detail below. The embodiments described below are exemplary and are merely intended to explain the present disclosure but should not be construed as a limitation to the present disclosure.

The present disclosure provides a synchronous sequencing method, a method for correcting a base-calling result of synchronous sequencing, a synchronous sequencing system, and a computer program product, each of which is described in detail below.

### Synchronous Sequencing Method

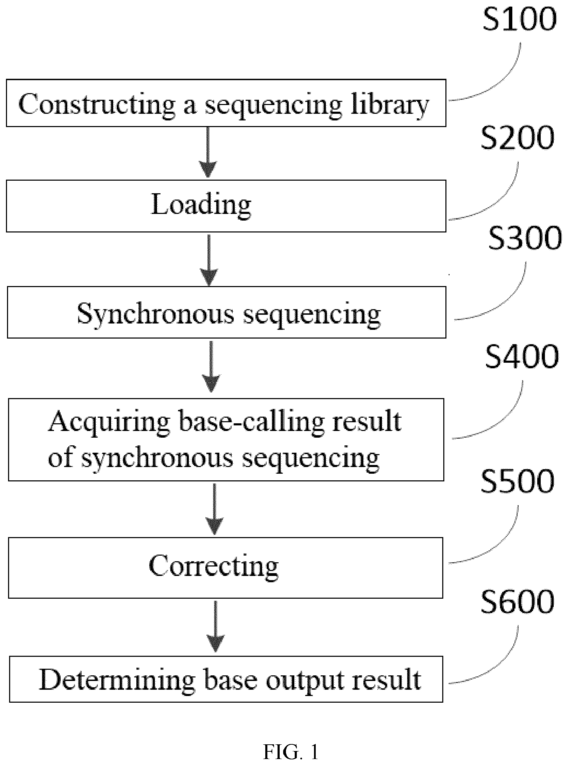

In an aspect of the present disclosure, a synchronous sequencing method is provided. According to an embodiment of the present disclosure, with reference to FIG. 1, the method includes:

S100: Construction of Sequencing Library

In this step, a sequencing library is constructed for a nucleic acid sample to be tested.

S200: Loading

In this step, the sequencing library is loaded onto a sequencing chip. The sequencing chip is provided with at least one composite template sample spot, and the composite template sample spot is provided with at least one sequencing template.

As used in the present disclosure, the term "composite template" refers to include at least one sequencing template obtained by amplification in advance, with no strict limitations on the amplification method, which can be rolling circle amplification, bridge amplification, etc.. For rolling circle amplification, the copy number variations of the different sequencing templates or the concentration variations of the sequencing primers for the plurality of sequencing templates can be controled to regulate the signal quantities generated by different sequencing templates. Specifically, the duration of polymerization extension reaction during sequencing library construction can be controled to regulate the copy number variations of the sequencing templates. For bridge amplification, the signal quantities generated by different sequencing templates can be distinguished using the following two methods. Method (1): Modifying the amplification primers on the surface of the chip. One type of amplification primer does not contain a cleavable group, and the other type of primer is mixed with a certain proportion of primers with cleavable groups (e.g., enzyme-cleavable or photo-cleavable). After amplification, removing copies containing the cleavable group. Thus, the control of the signal quantity relationship between the two DNA strands can be achieved. Method (2): During the sequencing process, controlling the concentration ratio of two sequencing primers, specifically by introducing a reversible blocking group into one of the sequencing primers.

In the present disclosure, the number of sequencing templates for synchronous sequencing is not strictly limited. Generally, at least two templates are required for sequencing at one position, namely a nucleic acid to be tested and a label sequence. The label sequence serves as a label for identification in a subsequent machine learning model. In the case of two label sequences, three templates are required.

In the present disclosure, the positions of the plurality of sequencing templates are also not strictly limited. They can be located at different positions on the same nucleic acid molecule, on different nucleic acid molecules, at different positions on the same DNA nanoball, or on different strands of the same DNA nanoball, which can be flexibly selected according to actual situations.

S300: Synchronous Sequencing

In this step, a plurality of synchronous sequencing reaction cycles is performed on the sequencing library. An image set generated in each of the plurality of synchronous sequencing reaction cycles constitutes a raw image set of the synchronous sequencing. Specifically, in synchronous sequencing, a plurality of composite template sample spots is provided, at least one sequencing template is provided in the composite template sample spot, with a plurality of sequencing reaction cycles being performed on the multiple sequencing templates simultaneously, and each of the plurality of synchronous sequencing reaction cycles generates a respective image.

The specific method of synchronous sequencing used in the present disclosure is well known in the art, with no strict limitations in the present disclosure.

According to an embodiment of the present disclosure, signal quantities generated by the plurality of sequencing templates follow a predetermined relationship. By predetermining variations in the signal quantities generated by different sequencing templates, the corresponding sequencing template can be identified for each base being sequenced. Specifically, the predetermining method includes controlling the copy number variations of the plurality of sequencing templates or controlling the concentration variations of the sequencing primers for the plurality of sequencing templates.

S400: Acquisition of Base-Calling Result of Synchronous Sequencing

In this step, a base-calling result of the synchronous sequencing is acquired based on the raw image set of the synchronous sequencing. The base-calling result includes a signal intensity value of each base channel in each of the plurality of synchronous sequencing reaction cycles.

S500: Correction

In this step, the signal intensity value of each base channel is corrected based on a predetermined correction parameter to obtain a corrected base-calling result. The correction parameter includes at least one of a crosstalk correction parameter and a phasing correction parameter for each base channel. Thus, the influences of crosstalk interference and phasing interference are avoided after eliminating noise in the early stage.

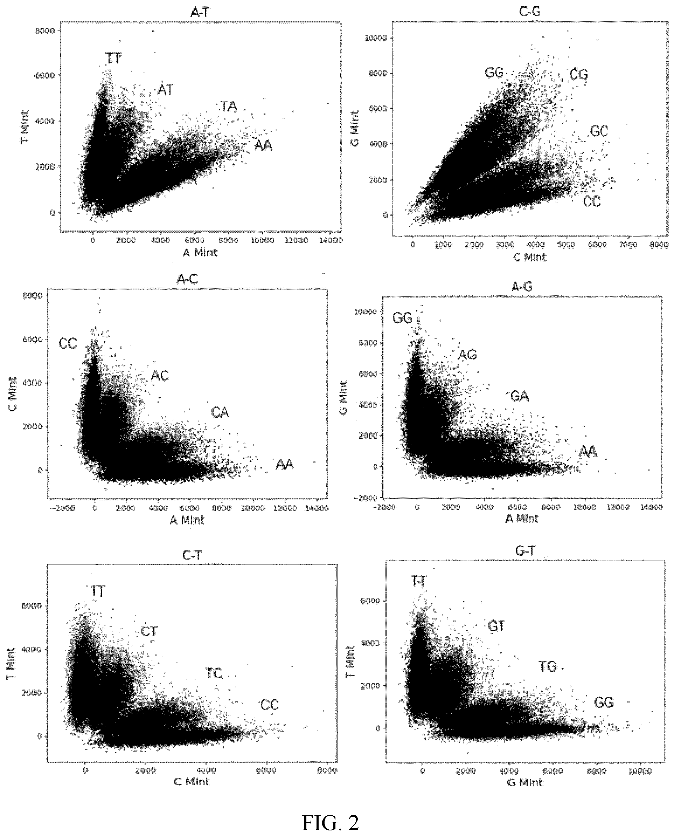

FIG. 2 shows scatter plots of initial signals between every two of four channels under synchronous sequencing conditions. Based on the principle of synchronous sequencing, two bases from different sources emit fluorescence of each nanoball in each cycle. In the synchronous sequencing mode, the four types of bases can collectively form 16 base combinations: AA, CA, TA, GA, CC, GC, TC, GC, GG, CG, TG, AG, TT, AT, CT, and GT. The crosstalk diagrams, which show scatter plots of fluorescence intensity between each pair of channels, display four distinct arms under synchronous sequencing conditions. For instance, in the A-T plot, from top to bottom, the base combinations TT, AT, TA, and AA appear in order, where the second-highest signal precedes the highest signal. Due to significant optical crosstalk between A and T bases and between C and G bases, the angle between the outer arms in these channel pairs is less than 90 degrees. In contrast, the outer arms between other channel pairs are nearly orthogonal. As a result, the presence of optical crosstalk leads to inaccuracies in signal detection across base channels. This deviation on synchronous sequencing can be particularly significant, which results in an inaccurate distinction between two bases, thereby possibly rendering the sequencing results unusable.

Under synchronous sequencing conditions, the signals from the previous or subsequent cycle's four channels may interfere with the signals of the current cycle's four channels, either in advance (leading) or with a delay (lagging). Therefore, phase correction of the current cycle's signals is required based on the sequencing signals from the previous or subsequent cycle.

Accordingly, signal intensity values of each base channel are corrected based on the crosstalk correction parameter and/or phase correction parameter of each base channel, in order to eliminate noise and enable base-calling of two bases per cycle in synchronous sequencing.

According to an embodiment of the present disclosure, the correction parameter is determined by: identifying a plurality of high-confidence composite sample spots among the plurality of composite template sample spots based on the base-calling result; identifying, for a given base channel, a plurality of first reference sample spots and a plurality of second reference sample spots among the plurality of high-confidence composite sample spots, wherein the plurality of first reference sample spots are selected from crosstalk correction parameter reference sample spots and the plurality of second reference sample spots are selected from phasing correction parameter reference sample spots; and identifying, for the given base channel, the crosstalk correction parameter of the given base channel based on a base-calling result of the plurality of first reference sample spots and the phasing correction parameter of the given base channel based on a base-calling result of the plurality of second reference sample spots.

The "high-confidence composite sample" described in the present disclosure refers to a collection of multiple high-confidence samples. A high-confidence sample mainly refers to a sample that is subject to minimal signal interference. Due to the significant optical crosstalk between bases A and T, as well as between bases C and G, sample spots where the base-calling results indicate only one type of base can be considered as high-confidence sample spots in a given sequencing reaction cycle, such as AA, TT, GG, and CC. This ensures the accuracy of the determined correction parameters.

According to an embodiment of the present disclosure, for the given base channel, the crosstalk correction parameter sample spots include a composite sample spot satisfying the following condition: in the given sequencing reaction cycle, the base-calling result of the composite sample spot indicates only one type of base that is different from the given base. This effectively prevents significant optical crosstalk between bases A and T and between bases C and G from adversely affecting the identification of the crosstalk correction parameters.

According to an embodiment of the present disclosure, the crosstalk correction parameter is obtained by training the following formula with a signal intensity value of each base channel from the plurality of first reference sample spots:

$$yi_{(B1,N)} = \beta 0 + \beta 1 * Xi_{(B2,N)} + \beta 2 * Xi_{(B3,N)} + \beta 3 * Xi_{(B4,N)} + \in$$

where: B1, B2, B3, and B4 represent one of base A channel, base T channel, base G channel, and base C channel, respectively, with B1 representing the given base channel; N represents the serial number of the given cycle; $yi_{(B1,N)}$ represents the signal intensity value of the given base channel in the given cycle N; $Xi_{(B2,N)}$, $Xi_{(B3,N)}$, and $Xi_{(B4,N)}$ represent signal intensity values of given base channels B2, B3, and B4 in the given cycle N, respectively; $\beta 0$, $\beta 1$, $\beta 2$, and $\beta 3$ represent crosstalk correction parameters for the given base channel; and $\in$ represents an error parameter. The formula is trained with a regression model.

The specific type of the regression model is not strictly limited in the present disclosure and may be, for example, Logistic Regression (LR), Linear Regression, Multiple Linear Regression (MLR) model, Generalized Linear Model (GLM), etc. Since the regression model is simple, highly interpretable, and easy to implement, it is widely applied in fields such as machine learning and deep learning.

To calculate the impact factor of noise in each channel, it is necessary to first select appropriate spots. Taking the calculation of the C channel as an example, the signal in the non-emission state of the C channel is attributed to crosstalk from other channels as well as phasing effects from previous and subsequent cycles. Therefore, sample spots where the current cycle is identified as GG, AA, or TT are selected to calculate the crosstalk coefficient of each channel relative to C.

$$yi_{(C,N)} = \beta 0 + \beta 1 * Xi_{(A,N)} + \beta 2 * Xi_{(G,N)} + \beta 3 * Xi_{(T,N)} + \in$$

$\beta 1$, $\beta 2$, and $\beta 3$ are the crosstalk coefficients of each channel relative to the C channel. The same applies to the calculation of other channels.

According to an embodiment of the present disclosure, the formula is trained with a regression model.

According to an embodiment of the present disclosure, for the given base channel, the plurality of second reference sample spots are composite sample spots that satisfy the following conditions: (A) in the given sequencing reaction cycle, the base-calling result of the composite sample spot indicates only one type of base that is different from the given base; and (B) in at least one of the previous or subsequent cycles of the given sequencing reaction cycle, the base-calling result of the composite sample spot indicates only one type of the given base.

According to an embodiment of the present disclosure, in condition (B), if in the previous cycle of the given sequencing reaction cycle, the base-calling result of the composite sample spot indicates only one type of the given base, the second reference sample spot is identified as a third reference sample spot, wherein the third reference sample spot is selected from lagging phasing correction parameters reference sample spot; or in condition (B), if in the subsequent cycle of the given sequencing reaction cycle, the base-calling result of the composite sample spot indicates only one type of the given base, the second reference sample spot is identified as a fourth reference sample spot, wherein the fourth reference sample spot is selected from leading phasing correction parameters reference sample spot.

According to an embodiment of the present disclosure, the phasing correction parameter further includes at least one of lagging phasing correction parameters and leading phasing correction parameters, and the phasing correction parameter is obtained by training the following formula with a signal intensity value of each base channel from the plurality of second reference sample spots:

$$yi_{(B1,M)} = \beta01 + B4 * Xi_{(B1,M-1)}$$

or,

$$yi_{(B1,M)} = \beta02 + B5 * Xi_{(B1,M+1)}$$

where: B1 represents the given base channel, M represents the serial number of the given cycle, M+1 represents the number of the subsequent cycle of the given cycle, M-1 represents the number of the previous cycle of the given cycle; β01 and B4 represent lagging phasing correction parameters for the given base channel, and β02 and B5 represent leading phasing correction parameters for the given base channel. The formula is trained with a regression model.

Taking the calculation of the C channel as an example, as previously described, the signal in the non-emission state of the C channel is attributed to crosstalk from other channels as well as Lagrunon (lagging phasing and leading phasing) effects from previous and subsequent cycles. To calculate the phasing coefficient of each channel relative to the C channel, sample spots where the current cycle is identified as GG, AA, or TT are selected. The calculation of the phasing coefficients involves the signal values of the previous and subsequent cycles, while ensuring minimal interference from the current cycle. For lagging phasing, sample spots are selected where current cycle (N) is identified as AA or TT, previous cycle (N-1) is identified as CC, and subsequent cycle (N+1) is identified as AA or TT, to calculate the lagging coefficient for the C channel. Similarly, for leading phasing, sample spots are selected where current cycle (N) is pre-called as AA or TT, previous cycle (N-1) is identified as AA or TT, and subsequent cycle (N+1) is identified as CC, to calculate the Lagrunon coefficient for the C channel. The same applies to the calculation of other channels.

$$yi_{(C,N)} = \beta0 + \beta4 * Xi_{(C,N-1)}$$

$$yi_{(C,N)} = \beta0 + \beta5 * Xi_{(C,N+1)}$$

$\beta4$ and $\beta5$ are the phasing coefficients of the C channel in the previous and subsequent cycles, respectively. The same applies to the calculation of other channels.

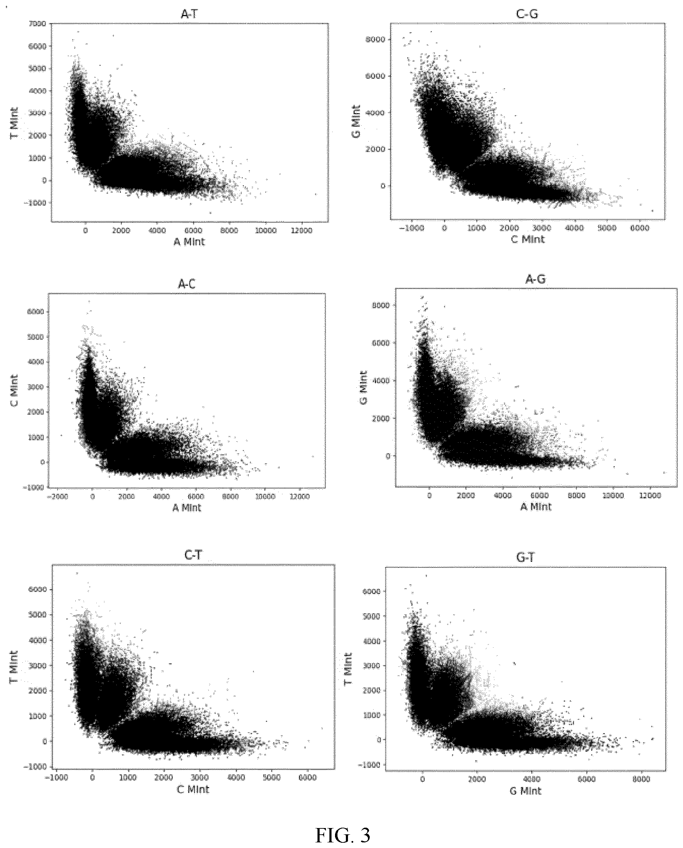

As can be seen from FIG. 3, after correcting the base-calling result of the synchronous sequencing as described above, noise can be effectively eliminated, and the signal distributions between channels form perpendicular angles. The separation between the arms is clearly defined. The correction process eliminates the influence between cycles and signals, resulting in more independent data between channels in each cycle. This enhances the authenticity of the data and facilitates subsequent model training.

According to an embodiment of the present disclosure, the method further includes inputting the corrected base-calling result as an input feature into a machine learning model to output a base combination of synchronous sequencing.

According to an embodiment of the present disclosure, the machine learning model is trained in a supervised manner using a reference sequence with a predetermined sequence as a training set. The reference sequence is subjected to synchronous sequencing, and a base-calling result obtained from a raw image of synchronous sequencing is corrected to generate a corrected base-calling result of each cycle as an input feature. The reference sequence is subjected to synchronous sequencing, and a base-calling result obtained from a raw image of synchronous sequencing is corrected to generate a corrected base-calling result of each cycle as an input feature. The base combination in the reference sequence corresponding to the base-calling result of each cycle is used as a label.

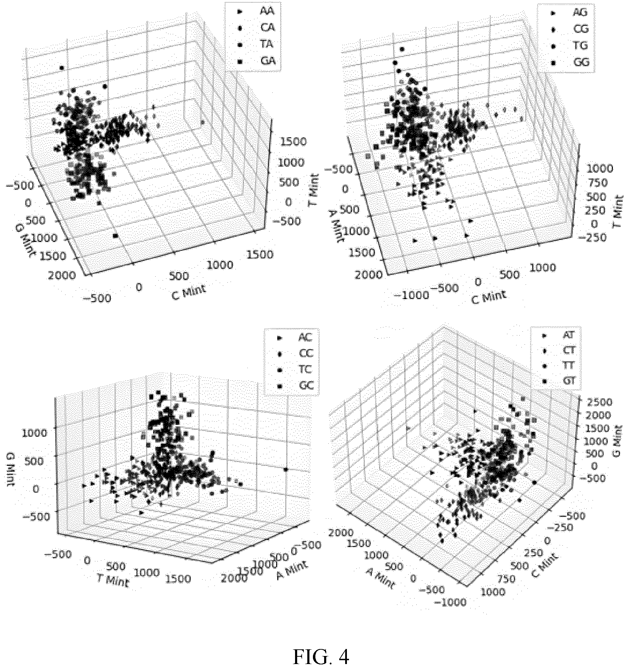

After the aforementioned noise elimination to avoid crosstalk and phase interference, the corrected base-calling results are normalized using min-max normalization. The signal channel emitting the strongest fluorescence in each base combination is identified, and the data are divided into four categories according to the maximum signal. As shown in FIG. 4, the four categories NA, NC, NG, and NT correspond to A, C, G, and T as the dominant signal base, respectively. Taking NA as an example, the strongest signal is in the A channel, while the second-strongest signal can be in any of the A, C, G, or T channels. By plotting the signals from the three non-A channels, four distinct base combinations can be visualized: the cluster at the origin represents AA, the arm along the C-axis represents CA, the arm in the G-axis direction represents GA, and the arm along the T-axis represents TA. Apart from the origin, the other spots are tightly clustered with blurred boundaries, making it difficult to distinguish between combinations. Moreover, when the second-strongest signal is weak, it may be confused with background fluorescence, which affects the accuracy of base combination determination. Therefore, it is necessary to apply a pre-trained machine learning model to the corrected base-calling results in order to accurately determine the base combinations.

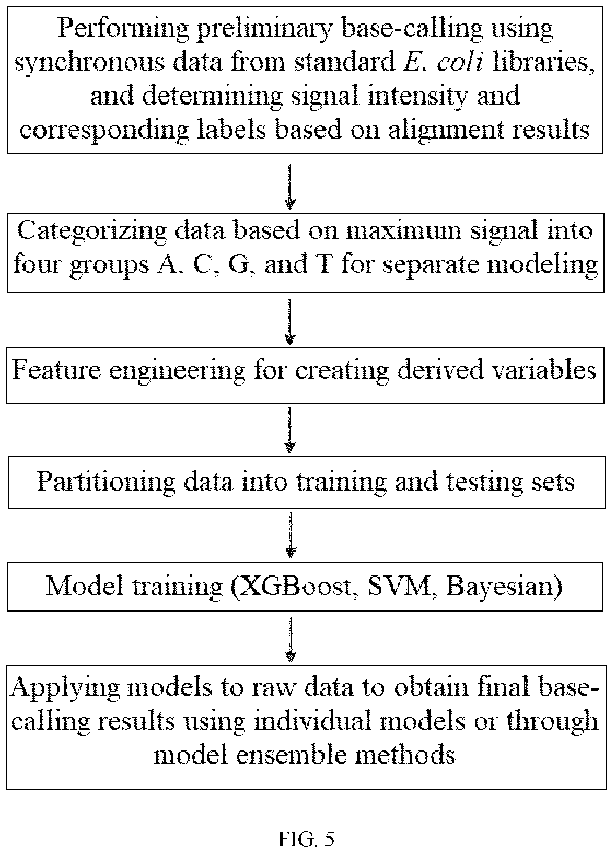

FIG. 5 shows a flowchart for constructing a machine learning model. First, to establish a supervised learning model, a label is required for training data. In the context of genomic sequencing data, labels are obtained by aligning the sequencing data to a reference genome to retrieve the accurate sequence signals. In this embodiment, the reference genome used for sequencing is a standard *E. coli* library. Sequencing data generated from synchronous sequencing of the standard *E. coli* library is first corrected, then a preliminary base-calling ("pre-call") is performed based on the maximum and second-highest signal intensities and a predefined threshold for their signal ratio. The corrected results are aligned with the reference genome to generate final labels. For each DNB (DNA nanoball) in each sequencing cycle, the corrected signal values and their corresponding labels are extracted.

Subsequently, based on the maximum signal channel, the sequencing data are modeled separately for the four bases A, C, G, and T. Taking the NA model as an example, the model is trained using signals corresponding to the labels AA, GA, CA, and TA within the aligned data. Derived variables are constructed based on the original four-channel signal intensities, extracting features across multiple dimensions. This process constitutes the feature engineering stage, including feature selection to remove redundant variables and retain those with the highest correlation to model accuracy. The dataset is then split into training and testing sets, and model accuracy is validated using cross-validation. The trained model is applied to an untrained validation dataset. The base-calling performance is evaluated by comparing results with the reference genome using metrics such as mapping rate and error rate. This training approach results in four independent models for predicting A, C, G, and T respectively, using the same set of training features. The final base-calling results can be determined by using any single model or an ensemble (e.g., several in the table) of models. If multiple models are used, the predictions are integrated to provide the final result.

According to an embodiment of the present disclosure, the machine learning model is at least one of Bayesian, Support Vector Machines (SVM), k-Nearest Neighbors (KNN), Random Forest, XGBoost, and Neural Network. The above models may be used independently or in combination as ensemble models. From a modeling perspective, the SVM model is based on distance calculations and requires normalization, and it is computationally intensive. Models such as XGBoost, Random Forest, and AdaBoost are tree-based ensemble methods rooted in probabilistic statistics. Bayesian classifiers also use probabilistic statistics, but their simplicity may result in lower accuracy compared to other ensemble models. As shown in the table below, among the tested machine learning models, Random Forest achieves the highest accuracy:

|  | Random Forest | XGBoost | SVM | Bayesian |
|---|---|---|---|---|
| Accuracy | 0.980 | 0.978 | 0.900 | 0.900 |
| Recall | 0.977 | 0.980 | 0.880 | 0.890 |
| F1-Score | 0.977 | 0.978 | 0.880 | 0.890 |

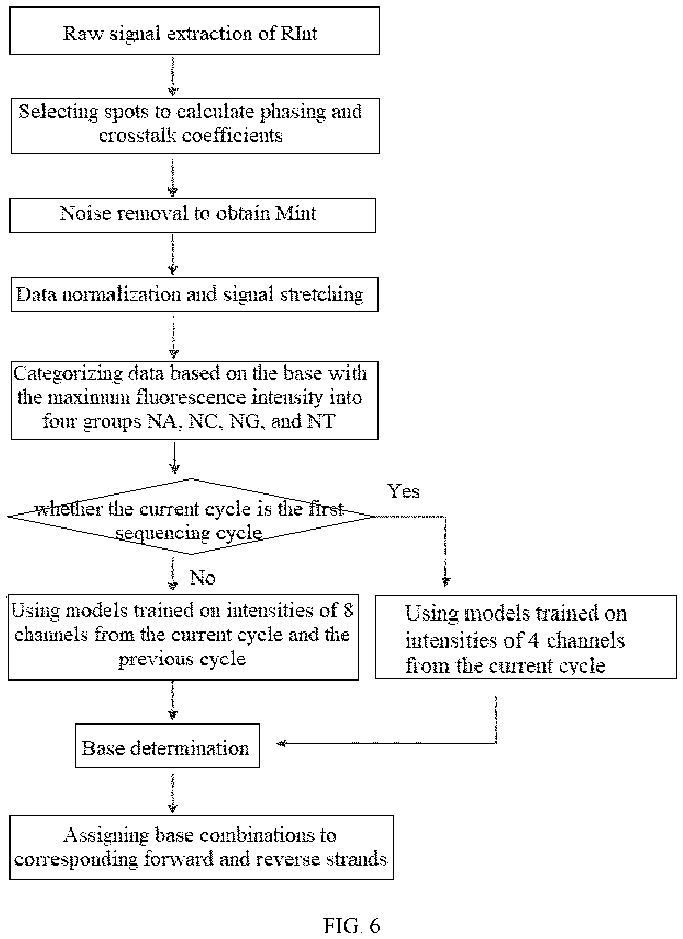

FIG. 6 shows the main steps of base calling under synchronous sequencing, where "RInt" refers to the raw signal intensity values extracted from the sequencing image, "Mint" refers to the corrected signal values after applying crosstalk and phasing corrections, "Intensity" refers to the measured signal values (e.g., fluorescence intensity). First, the raw extracted signal (RInt) is acquired. Based on the signal intensity values, the phasing coefficient and the crosstalk coefficient are determined, and the signal intensity value of each base channel is corrected to eliminate the noise and obtain Mint. The above pre-processed data are subjected to normalization/signal stretching processing to identify the maximum luminescent base signal, and divided into four categories of NA, NG, NC, and NT according to the maximum signal. Depending on whether the cycle is the first sequencing cycle, the selection of the machine learning model varies. Specifically, for the first sequencing cycle, the current cycle's signal values and their derived variables are used. These derived variables may be generated from the initial four-channel fluorescence intensities, for example: the intensity ratio of base A to base T, the difference between the maximum and second-highest intensity values, the signal-to-noise ratio between the maximum intensity and background signal, etc. For all subsequent cycles beyond the first, the model inputs are constructed using both the previous and current cycles' signal values, including eight channels in total, along with their derived variables. These features are then input into a training model. Finally, the identified base combination results are output in the standard paired-end sequencing format, including a single-read FASTQ file and a paired-read FASTQ file.

S600: Determination of Output Base Result of Synchronous Sequencing

In this step, based on the corrected base-calling result, a base result output of synchronous sequencing is determined. Specifically, the base results may include AA, CA, TA, GA, CC, GC, TC, GC, GG, CG, TG, AG, TT, AT, CT, and GT.

## Method for Correcting Base-Calling Result of Synchronous Sequencing

In yet another aspect of the present disclosure, a method for correcting a base-calling result of synchronous sequencing is provided. According to an embodiment of the present disclosure, the method includes: acquiring a raw image set of the synchronous sequencing, wherein in the synchronous sequencing, at least one composite template sample spot is provided, at least one sequencing template is provided in the composite template sample spot, with a plurality of sequencing reaction cycles being performed on the at least one sequencing template, and an image set generated in each of the plurality of sequencing reaction cycles constitutes the raw image set; acquiring a base-calling result of the synchronous sequencing based on the raw image set, wherein the base-calling result includes a signal intensity value of each base channel in each of the plurality of sequencing reaction cycles; and correcting the signal intensity value of each base channel based on a predetermined correction parameter to obtain a corrected base-calling result, wherein the correction parameter includes at least one of a crosstalk correction parameter and a phasing correction parameter for each base channel.

It should be noted that the features and advantages described above with respect to the synchronous sequencing method are equally applicable to the method for correcting a base-calling result of synchronous sequencing, and are not repeated here for brevity.

## Synchronous Sequencing System, Electronic Device, And Computer-Readable Storage Medium

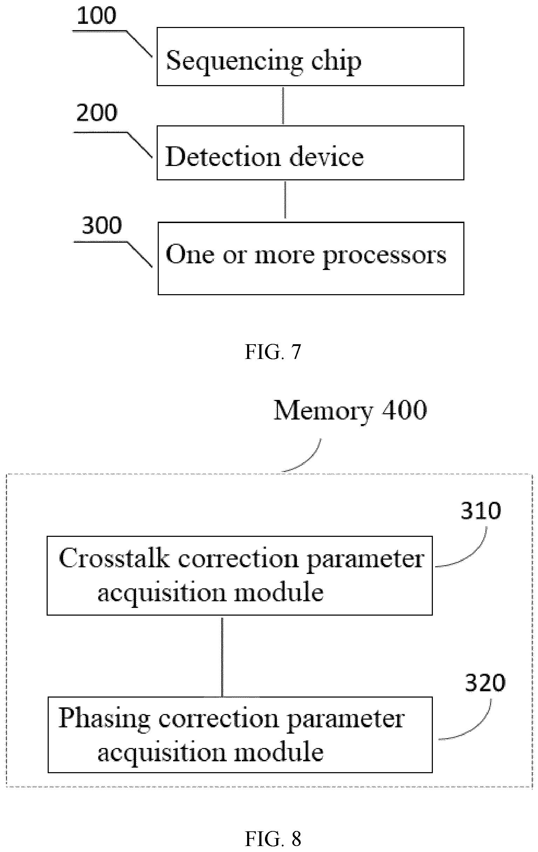

In yet another aspect of the present disclosure, a synchronous sequencing system is provided. According to an embodiment of the present disclosure, with reference to FIG. 7, the synchronous sequencing system includes: a sequencing chip 100, a detection device 200, and one or more processors 300, each of which is described in detail below.

According to an embodiment of the present disclosure, the sequencing chip 100 is provided with at least one composite template sample spot, and the composite template sample spot is provided with at least one sequencing template.

According to an embodiment of the present disclosure, the detection device 200 is configured to perform a plurality of synchronous sequencing reaction cycles on a sequencing library, wherein an image set generated in each of the plurality of synchronous sequencing reaction cycles constitutes a raw image set of the synchronous sequencing.

According to an embodiment of the present disclosure, the detection device may be a sequencer configured to perform next-generation sequencing (NGS). In an embodiment, the sequencer is configured to perform sequencing-by-synthesis (SBS) using reversible dye terminators for high-throughput parallel sequencing. In another embodiment, the sequencer is configured to perform sequencing-by-ligation.

According to an embodiment of the present disclosure, one or more processors 300 are configured to execute: (A) acquiring a base-calling result of the synchronous sequencing based on the raw image set of the synchronous sequencing, wherein the base-calling result includes a signal intensity value of each base channel in each of the plurality of synchronous sequencing reaction cycles, (B) correcting the signal intensity value of each base channel based on a predetermined correction parameter to obtain a corrected base-calling result, wherein the correction parameter includes at least one of a crosstalk correction parameter and a phasing correction parameter for each base channel, and (C) determining a base output result of the synchronous sequencing based on the corrected base-calling result.

According to an embodiment of the present disclosure, one or more processors 300 are further configured to execute functional modules: a crosstalk correction parameter acquisition module 310 and a phasing correction parameter acquisition module 320. Specifically, with reference to FIG. 8, the synchronous sequencing system may also have one or more memories 400 with an optional computer program stored thereon. The computer program may include a crosstalk correction parameter acquisition module 310 and a phasing correction parameter acquisition module 320.

According to an embodiment of the present disclosure, the crosstalk correction parameter acquisition module 310 is used to obtain the crosstalk correction parameter by training the following formula with the signal intensity value of each base channel from the plurality of first reference sample spots:

$$yi_{(B1,N)} = \beta 0 + \beta 1 * Xi_{(B2,N)} + \beta 2 * Xi_{(B3,N)} + \beta 3 * Xi_{(B4,N)} + \in$$

where: B1, B2, B3, and B4 represent one of base A channel, base T channel, base G channel, and base C channel, respectively, with B1 representing the given base channel; N represents the serial number of the given cycle; $yi_{(B1,N)}$ represents the signal intensity value of the given base channel in the given cycle N; $Xi_{(B2,N)}$, $Xi_{(B3,N)}$, and $Xi_{(B4,N)}$ represent signal intensity values of given base channels B2, B3, and B4 in the given cycle N, respectively; $\beta0$, $\beta1$, $\beta2$, and $\beta3$ represent crosstalk correction parameters for the given base channel; and $\in$ represents an error parameter.

According to an embodiment of the present disclosure, the phasing correction parameter acquisition module 320 is used to obtain the phasing correction parameter by training the following formula with the signal intensity value of each base channel from the plurality of second reference sample spots:

$$\mathrm{yi}_{(\mathrm{B1,M})} = \ \beta01 + \mathrm{B4} * \mathrm{Xi}_{(\mathrm{B1,M-1})}$$

or,

$$\mathrm{yi}_{(\mathrm{B1,M})} = \ \beta02 + \mathrm{B5} * \mathrm{Xi}_{(\mathrm{B1,M+1})}$$

where: B1 represents the given base channel, M represents the serial number of the given cycle, M+1 represents the number of the subsequent cycle of the given cycle, M-1 represents the number of the previous cycle of the given cycle, $\beta01$ and B4 represent lagging phasing correction parameters for the given base channel, and $\beta02$ and B5 represent leading phasing correction parameters for the given base channel.

One or more processors 300 may be general-purpose or dedicated processors. For example, the processor may be a baseband processor or a central processing unit (CPU). The baseband processor can be used to process communication protocols and communication data. The CPU may be used to control communication devices (e.g., base stations, baseband chips, terminal devices, terminal device chips, distributed units (DU), or centralized units (CU)), execute computer programs, and process data associated with computer programs. The processor 300 may be implemented on an integrated circuit (IC), an analog IC, a radio-frequency integrated circuit (RFIC), a mixed-signal IC, an application-specific integrated circuit (ASIC), a printed circuit board (PCB), or other electronic components. The processor and the transceiver may be fabricated using various IC process technologies, such as complementary metal-oxide-semiconductor (CMOS), n-type metal-oxide-semiconductor (NMOS), p-type metal-oxide-semiconductor (PMOS), bipolar junction transistor (BJT), bipolar CMOS (BiCMOS), silicon-germanium (SiGe), or gallium arsenide (GaAs).

It will be appreciated by those skilled in the art that the various illustrative logical blocks and steps described in the embodiments of the present application may be implemented in electronic hardware, computer software, or a combination of both. Whether such functionality is implemented in hardware or software depends on the particular application and the overall system design requirements. For each specific application, different approaches may be employed by those skilled in the art to achieve the described functionality, without departing from the scope of protection defined by the embodiments of this application.

In the above embodiments, the methods may be implemented in whole or in part using software, hardware, firmware, or any combination thereof. When implemented in software, the methods may be implemented in whole or in part in the form of a computer program product. A computer program product includes one or more computer programs, which, when loaded and executed on a computer, result in the execution of all or part of the processes or functionalities described in the embodiments of the present application. The computer may be a general-purpose computer, a dedicated computer, a computer network, or any other programmable device. The computer programs may be stored on a computer-readable storage medium or transmitted from one computer-readable medium to another. For example, the computer programs may be transmitted from one website, computer, server, or data center to another via wired means (e.g., coaxial cable, fiber optics, digital subscriber line (DSL)) or wireless means (e.g., infrared, radio, microwave, etc.).

To this end, in yet another aspect of the present disclosure, a computer-readable storage medium is provided. According to an embodiment of the present disclosure, the computer-readable storage medium has one or more programs stored thereon that are executable by one or more processors to implement the method for correcting a base-calling result of synchronous sequencing. A computer-readable storage medium may refer to any available medium that can be accessed by a computer, or a data storage device such as a server or data center that integrates one or more such available media. Available media may include magnetic media (e.g., floppy disks, hard disks, magnetic tapes), optical media (e.g., digital video discs (DVDs)), or semiconductor media (e.g., solid state disks (SSDs)).

In yet another aspect of the present disclosure, an electronic device is provided. According to an embodiment of the present disclosure, the electronic device includes a memory and a processor. The memory has a program stored thereon that is executable by the processor, and the program, when executed by the processor, implements the method for regional partitioning of in situ sequencing results. Specifically, the electronic device may be any intelligent terminal, including, for example, tablet computers, computing clusters, sequencing instruments, or vehicle-mounted computers.

As used in the present disclosure, the term "memory" refers to any computer program product, device, and/or apparatus (e.g., disks, optical disks, memory, programmable logic devices (PLDs)) used to provide instructions and/or data to a programmable processor. This includes machine-readable media that receive machine-readable instructions. The memory may be implemented as Read-Only Memory (ROM), static storage, dynamic storage, or Random Access Memory (RAM). The memory may store the operating system and other application programs. When the technical solutions of the embodiments of the present application are implemented via software or firmware, the relevant program code is stored in the memory and executed by the processor to perform the training method of a gene sequencing model or a gene sequencing method in accordance with the embodiments of the present application. Specifically, the memory includes a crosstalk correction parameter acquisition module and a phasing correction parameter acquisition module.

In an embodiment, the electronic device may further include input/output interfaces, communication interfaces, and a bus. The input/output interface is configured to facilitate the input and output of information. The communication interface is configured to enable communication and interaction between the device and other devices, which can be realized through wired means (e.g., USB, Ethernet) or wireless means (e.g., mobile networks, Wi-Fi, Bluetooth). The bus is configured to transfer information among various components of the device (e.g., processor, memory, input/output interface, and communication interface).

It should be noted that the in situ sequencing method described above may also be implemented using the in situ sequencing system. The features and advantages described with respect to the in situ sequencing method are equally applicable to the in situ sequencing system, the electronic device, and the computer-readable storage medium, and are not repeated here for brevity.

It should further be noted that the features and advantages described with respect to the synchronous sequencing method and the method for correcting a base calling result of synchronous sequencing are likewise applicable to the synchronous sequencing system, the electronic device, and the computer-readable storage medium, and are not repeated here for brevity.

The following examples are provided to further illustrate the disclosed solutions. It will be understood by those skilled in the art that the following embodiments are intended for illustrative purposes only and should not be construed as limiting the scope of the present disclosure. Unless otherwise specified, the technical means or conditions employed in the embodiments shall be those described in the literature in the field or those provided in product manuals. Reagents or instruments for which the manufacturer is not specified are commercially available conventional products.

1. Equipment:

MGISEQ-2000 sequencer, MGISEQ-2000 sequencing reagent slide (715 nm), mini DNB loading apparatus, PCR instrument, PCR 8-tube strips, pipette set, high-speed centrifuge, mini centrifuge, and vortex mixer.

2. Reagents:

Table 1:

| Reagent Name | Brand |
| --- | --- |
| DNA Nanoball Preparation Buffer | MGI |
| PNK Enzyme (Polynucleotide Kinase) | MGI |
| T4PNK 10X Reaction Buffer | MGI |
| MGISEQ-2000RS High-Throughput Sequencing Reagent | MGI |
| DNA Nanoball Preparation Enzyme Mix I | MGI |
| DNA Nanoball Preparation Enzyme Mix II | MGI |
| LTE Buffer | MGI |
| DNA Nanoball Termination Buffer | MGI |
| DNA Nanoball Loading Buffer IV | MGI |
| 5XSSC Buffer | MGI |
| *Escherichia Coli* Standard Library | MGI |
| 10x Phi29 Buffer | BGI Research Institute |
| First-Strand Sequencing Primer with Linker IP1-X1 Powder | Sangon |
| First-Strand Sequencing Primer with Linker IP1-X2 Powder | Sangon |

(continued)

| Reagent Name | Brand |
|---|---|
| Second-Strand Sequencing Primer IP3 | MGI |
| DNB Read Buffer (Reb) | MGI |
| EDTA | - |
| Formamide | - |

3. Primer sequences:

First-strand sequencing primer with linker IP1-x1:

CGCCG ACGCA CAGGG TGCCT CGACC GCATG GCGCG GAACC ATGGT TCCGC GCCAA CTCCT TGGCT CACAG AACGA CATGG CTACG ATCCG ACTT (94nt, SEQ ID NO: 1).

First-strand sequencing primer with linker IP1-x2:

CATGC GGTCG AGGCA CCCTG TGCGT CGGCG GGCTG CATGC CGGCA TGCAG CCCAA CTCCT TGGCT CACAG AACGA CATGG CTACG ATCCG ACTT (94nt, SEQ ID NO: 2).

First-strand sequencing primer with linker and 3' blocking IP1-x1-OP:

CGCCG ACGCA CAGGG TGCCT CGACC GCATG GCGCG GAACC ATGGT TCCGC GCCAA CTCCT TGGCT CACAG AACGA CATGG CTACG ATCCG ACTT (94nt, SEQ ID NO: 1).

First-strand sequencing primer with linker and 3' blocking IP1-x2-OP:

CATGC GGTCG AGGCA CCCTG TGCGT CGGCG GGCTG CATGC CGGCA TGCAG CCCAA CTCCT TGGCT CACAG AACGA CATGG CTACG ATCCG ACTT (94nt, SEQ ID NO: 2).

4. Reagent Preparation

1) Primer Dissolution
A 1.5-milliliter centrifuge tube containing primer powder was centrifuged at the maximum speed in an Eppendorf high-speed centrifuge (5415D) for 5 minutes. The primers were dissolved in ultrapure water in accordance with the instructions on the primer label to prepare a 100 $\mu$M stock solution.
2) The preparation of 1 $\mu$M working solution of the first-strand sequencing primer with linker, IP1-xlinker, is shown in Table 2.

Table 2:

| Reagent Name | Volume | Final Concentration |
|---|---|---|
| 100 μM First-Strand Sequencing Primer with Linker IP1-x1 Stock Solution | 50 μL | 0.5 μM |
| 100 μM First-Strand Sequencing Primer with Linker IP1-x2 Stock Solution | 50 μL | 0.5 μM |
| 10X Phi29 Buffer | 1 mL | 1X |
| Ultrapure Water | 8.9 mL | --- |
| Total | 10 mL | --- |

3) The preparation of DNB loading buffer V is shown in Table 3.

Table 3:

| Reagent Name | Volume | Final Concentration |
|---|---|---|
| DNB Loading Buffer IV | 100 μL | --- |
| EDTA (0.5 M) | 17 μL | --- |
| Total | 117 μL | --- |

4) The preparation of PNK enzyme reagent is shown in Table 4.

Table 4:

| 10U T4PNK (BGI) | 0.1U |
|---|---|
| T4PNK 10X Reaction Buffer (pH 5.9) | 1X |

5) The preparation of the mixed working solution of first- and second-strand sequencing primers, Insert Primer Mix, is shown in Table 5.

Table 5:

| Reagent Name | Volume | Final Concentration |
|---|---|---|
| 100 μM First-Strand Sequencing Primer with Linker IP1-x1 Stock Solution | 50 μL | 0.5 μM |
| 100 μM First-Strand Sequencing Primer with Linker IP1-x2 Stock Solution | 50 μL | 0.5 μM |
| 1.0 μM Second-Strand Sequencing Primer IP3 | 9.9 mL | 1.0 μM |
| Total | 10 mL | --- |

6) The preparation of the mixed working solution of first- and second-strand barcode primers, Barcode Primer Mix, is shown in Table 6.

Table 6:

| Reagent Name | Volume | Final Concentration |
|---|---|---|
| 100 μM First-Strand Barcode Primer BP1 Stock Solution | 100 μL | 1 μM |
| 100 μM Second-Strand Barcode Primer BP2 Stock Solution | 100 μL | 1 μM |
| 5X SSC Buffer | 9.8 mL | --- |
| Total | 10 mL | --- |

7) The preparation of 1 μM first-strand sequencing primer with linker and 3' phosphorylation working solution, IP1-xlinker, is shown in Table 7.

# EP 4 636 772 A1

Table 7:

| Reagent Name | Volume | Final Concentration |
|---|---|---|
| 100 μM First-Strand Sequencing Primer with Linker IP1-x1-OP Stock Solution | 50 μL | 0.5 μM |
| 100 μM First-Strand Sequencing Primer with Linker IP1-x2-OP Stock Solution | 50 μL | 0.5 μM |
| 10X Phi29 Buffer | 1 mL | 1X |
| Ultrapure Water | 8.9 mL | --- |
| Total | 10 mL | --- |

5. Sequencing Analysis Procedure

5.1 Preparation of DNB

DNA nanoballs (DNBs) were prepared using the *Escherichia coli* library by referring to the "MGISEQ-2000RS High-throughput Sequencing Reagent Kit User Manual". The volume of EII was adjusted to 1.6 μL, and the volume of the termination buffer was reduced by half by adding 10 μL of the termination buffer.

5.2 Loading DNB

An MGISEQ-2000 sequencing reagent slide was prepared. The DNBs were mixed uniformly with DNB loading buffer V at a volume ratio of 2:1, and then loaded onto the MGISEQ-2000 sequencer's sequencing reagent slide using a mini DNB loading apparatus.

5.3 Sequencing Procedure

A sequencing reagent kit was prepared by referring to the "MGISEQ-2000RS High-throughput Sequencing Reagent Kit User Manual". The reagent in well 13 was replaced with 1 μM of the first-strand sequencing primer working solution with Xlinker. The reagent in well 6 was replaced with DNB loading buffer IV. The reagent in well 7 was replaced with 1X phi29 buffer. The reagent in well 11 was replaced with REB reagent. The reagent in well 4 was replaced with formamide.
In accordance with the "MGISEQ-2000RS High-throughput Sequencing Reagent Kit User Manual", the sequencing reagent kit and the chip were placed into the MGI2000-RS sequencer. The corresponding script was selected, and PE5 was set for sequencing. To prove the feasibility of this scheme, the sequencing process was performed as follows: First, the second-strand sequencing primer was hybridized, followed by Cycle 5 sequencing of the second strand. Subsequently, the second-strand sequencing strand was blocked. Next, the first-strand sequencing primer was hybridized, followed by Cycle 5 sequencing of the first strand. After that, incubation with formamide was carried out to elute the sequencing strand from the template, thereby regenerating the template. Then, the first-strand sequencing primer was hybridized again, followed by Cycle 3 sequencing of the first strand. Finally, the second-strand sequencing primer was hybridized, followed by Cycle 3 sequencing of the second strand.

6. Sequencing Results and Analysis

The table below shows the raw signal intensity values of the first 10 cycles of a DNB.

| | Rin_A | Rin_C | Rin_G | Rin_T |
|---|---|---|---|---|
| Cycle 1 | -116 | 954 | 1412 | -80 |
| Cycle 2 | -91 | 1234 | 236 | -134 |
| Cycle 3 | 97 | -81 | -235 | 1323 |
| Cycle 4 | -224 | 641 | 1729 | -175 |
| Cycle 5 | 492 | -253 | -72 | 92 |
| Cycle 6 | 494 | -141 | -277 | 943 |
| Cycle 7 | 241 | 796 | -6 | 88 |
| Cycle 8 | -285 | 836 | 1130 | -216 |

(continued)

|  | Rin_A | Rin_C | Rin_G | Rin_T |
|---|---|---|---|---|
| Cycle 9 | 1 | 306 | -84 | 622 |
| Cycle 10 | -89 | 661 | 448 | -86 |

FIG. 2 shows scatter plots of initial signals between every two of four channels under synchronous sequencing conditions. Based on the principle of synchronous sequencing, two bases from different sources emit fluorescence of each nanoball in each cycle. In the synchronous sequencing mode, the four types of bases can collectively form 16 base combinations: AA, CA, TA, GA, CC, GC, TC, GC, GG, CG, TG, AG, TT, AT, CT, and GT. The crosstalk diagrams, which show scatter plots of fluorescence intensity between each pair of channels, display four distinct arms under synchronous sequencing conditions. For instance, in the A-T plot, from top to bottom, the base combinations TT, AT, TA, and AA appear in order, where the second-highest signal precedes the highest signal. Due to significant optical crosstalk between A and T bases and between C and G bases, the angle between the outer arms in these channel pairs is less than 90 degrees. In contrast, the outer arms between other channel pairs are nearly orthogonal. As a result, the presence of optical crosstalk leads to inaccuracies in signal detection across base channels. This deviation on synchronous sequencing can be particularly significant, which results in an inaccurate distinction between two bases, thereby possibly rendering the sequencing results unusable.

Under synchronous sequencing conditions, the signals from the previous or subsequent cycle's four channels may interfere with the signals of the current cycle's four channels, either in advance (leading) or with a delay (lagging). Therefore, phase correction of the current cycle's signals is required based on the sequencing signals from the previous or subsequent cycle.

Accordingly, signal intensity values of each base channel are corrected based on the crosstalk correction parameter and/or phase correction parameter of each base channel, in order to eliminate noise. Specifically, to calculate the impact factor of noise in each channel, it is necessary to first select appropriate spots. Taking the calculation of the C channel as an example, the signal in the non-emission state of the C channel is attributed to crosstalk from other channels as well as phasing effects from previous and subsequent cycles. Therefore, sample spots where the current cycle is identified as GG, AA, or TT are selected to calculate the crosstalk coefficient of each channel relative to C.

$$yi_{(C,N)} = \beta0 + \beta1 * Xi_{(A,N)} + \beta2 * Xi_{(G,N)} + \beta3 * Xi_{(T,N)} + \in$$

$\beta1$, $\beta2$, and $\beta3$ are the crosstalk coefficients of each channel relative to the C channel.

The calculation of the phasing coefficients involves the signal values of the previous and subsequent cycles, while ensuring minimal interference from the current cycle. Taking the C channel as an example, for lagging phasing, sample spots are selected where current cycle (N) is identified as AA or TT, previous cycle (N-1) is identified as CC, and subsequent cycle (N+1) is identified as AA or TT, to calculate the phasing coefficient for the C channel. Similarly, for leading phasing, sample spots are selected where current cycle (N) is pre-called as AA or TT, previous cycle (N-1) is identified as AA or TT, and subsequent cycle (N+1) is identified as CC.

$$yi_{(C,N)} = \beta0 + \beta4 * Xi_{(C,N-1)}$$

$$yi_{(C,N)} = \beta0 + \beta5 * Xi_{(C,N+1)}$$

$\beta4$ and $\beta5$ are the phasing coefficients of the C channel in the previous and subsequent cycles, respectively. The same applies to the calculation of other channels.

As can be seen from FIG. 3, after correcting the base-calling result of the synchronous sequencing as described above, noise can be effectively eliminated, and the signal distributions between channels form perpendicular angles. The separation between the arms is clearly defined. The correction process eliminates the influence between cycles and signals, resulting in more independent data between channels in each cycle and enhanced authenticity of the data. After the above noise elimination process, an intermediate signal, Mid Intensity, is obtained from the Raw signal. The specific data are shown in the table below.

|  | Min_A | Min_C | Min_G | Min_T |
|---|---|---|---|---|
| Cycle 1 | 169 | 194 | 1244 | -63 |

(continued)

|  | Min_A | Min_C | Min_G | Min_T |
|---|---|---|---|---|
| Cycle 2 | 128 | 1101 | -125 | -143 |
| Cycle 3 | 35 | -55 | -110 | 1352 |
| Cycle 4 | -51 | -180 | 1694 | -147 |
| Cycle 5 | 712 | -228 | 131 | -77 |
| Cycle 6 | 496 | -24 | -96 | 802 |
| Cycle 7 | 410 | 783 | -148 | -27 |
| Cycle 8 | -96 | 249 | 998 | -129 |
| Cycle 9 | 76 | 283 | -124 | 677 |
| Cycle 10 | 90 | 438 | 378 | -89 |

After the above noise elimination, the resulting data were normalized using min-max normalization. The signal channel emitting the strongest fluorescence in each base combination was identified, and the data were divided into four categories according to the maximum signal. Subsequently, the data were input into a pre-trained machine learning model (the training data and modeling workflow of the machine learning model are shown in FIG. 5), and the output prediction results were as follows. It can be seen that the method of the present disclosure enables accurate determination of base combinations in synchronous sequencing.

|  | Cycle 1 | Cycle 2 | Cycle 3 | Cycle 4 | Cycle 5 |
|---|---|---|---|---|---|
| Predicted Result | CG | CC | TT | GG | AA |
| True Result | CG | CC | TT | GG | AA |
|  | Cycle 6 | Cycle 7 | Cycle 8 | Cycle 9 | Cycle 10 |
| Predicted Result | AT | AC | CG | CT | GC |
| True Result | AT | AC | CG | CT | GC |

Although the embodiments of the present disclosure have been illustrated and described above, it should be understood that the above embodiments are exemplary and cannot be construed as limitations on the present disclosure. Changes, modifications, replacements, and variants to the above embodiments may be made by those skilled in the art within the scope of the present disclosure.

**Claims**

1.    A method of synchronous sequencing, comprising:

constructing a sequencing library for a nucleic acid sample to be tested;
loading the sequencing library onto a sequencing chip, wherein the sequencing chip is provided at least one composite template sample spot, the composite template sample spot being provided with at least one sequencing template;
performing a plurality of synchronous sequencing reaction cycles on the sequencing library, wherein an image set generated in each of the plurality of synchronous sequencing reaction cycles constitutes a raw image set of the synchronous sequencing;
acquiring a base-calling result of the synchronous sequencing based on the raw image set of the synchronous sequencing, wherein the base-calling result comprises a signal intensity value of each base channel in each of the plurality of synchronous sequencing reaction cycles;
correcting the signal intensity value of each base channel based on a predetermined correction parameter to obtain a corrected base-calling result, wherein the correction parameter comprises at least one of a crosstalk correction parameter and a phasing correction parameter for each base channel; and
determining a base output result of the synchronous sequencing based on the corrected base-calling result.

2. The method according to claim 1, wherein the plurality of sequencing templates is located at different positions on the same nucleic acid molecule.

3. The method according to claim 1, wherein the plurality of sequencing templates is located on different nucleic acid molecules.

4. The method according to claim 1, wherein signal quantities generated by the plurality of sequencing templates follow a predetermined relationship.

5. The method according to claim 1, wherein the correction parameter is determined by:

identifying a plurality of high-confidence composite sample spots among the plurality of composite template sample spots based on the base-calling result;
identifying, for a given base channel, a plurality of first reference sample spots and a plurality of second reference sample spots among the plurality of high-confidence composite sample spots, wherein the plurality of first reference sample spots are selected from crosstalk correction parameter reference sample spots and the plurality of second reference sample spots are selected from phasing correction parameter reference sample spots; and
identifying, for the given base channel, the crosstalk correction parameter of the given base channel based on a base-calling result of the plurality of first reference sample spots and the phasing correction parameter of the given base channel based on a base-calling result of the plurality of second reference sample spots.

6. The method according to claim 5, wherein the plurality of high-confidence composite sample spots comprises a composite sample spot where the base-calling result indicates only one type of base in a given sequencing reaction cycle.

7. The method according to claim 5, wherein for the given base channel, the plurality of first reference sample spots comprise a composite sample spot satisfying the following condition:
in the given sequencing reaction cycle, the base-calling result of the composite sample spot indicates only one type of base that is different from the given base.

8. The method according to claim 6, wherein the crosstalk correction parameter is obtained by training the following formula with a signal intensity value of each base channel from the plurality of first reference sample spots:

$$yi_{(B1,N)} = \beta0 + \beta1 * Xi_{(B2,N)} + \beta2 * Xi_{(B3,N)} + \beta3 * Xi_{(B4,N)} + \in$$

where:

B1, B2, B3, and B4 represent one of base A channel, base T channel, base G channel, and base C channel, respectively, with B1 representing the given base channel,
N represents the serial number of the given cycle,
$yi_{(B1,N)}$ represents the signal intensity value of the given base channel in the given cycle N,
$Xi_{(B2,N)}$, $Xi_{(B3,N)}$, and $Xi_{(B4,N)}$ represent signal intensity values of given base channels B2, B3, and B4 in the given cycle N, respectively,
$\beta0$, $\beta1$, $\beta2$, and $\beta3$ represent crosstalk correction parameters for the given base channel; and
$\in$ represents an error parameter; and
wherein the formula is trained with a regression model.

9. The method according to claim 5, wherein for the given base channel, the plurality of second reference sample spots are composite sample spots that satisfy the following conditions:

(A) in the given sequencing reaction cycle, the base-calling result of the composite sample spot indicates only one type of base that is different from the given base; and
(B) in at least one of the previous or subsequent cycles of the given sequencing reaction cycle, the base-calling result of the composite sample spot indicates only one type of the given base.

10. The method according to claim 9, wherein:

in condition (B), if in the previous cycle of the given sequencing reaction cycle, the base-calling result of the composite sample spot indicates only one type of the given base, the second reference sample spot is identified as a third reference sample spot, wherein the third reference sample spot is selected from lagging phasing correction parameters reference sample spot; or

in condition (B), if in the subsequent cycle of the given sequencing reaction cycle, the base-calling result of the composite sample spot indicates only one type of the given base, the second reference sample spot is identified as a fourth reference sample spot, wherein the fourth reference sample spot is selected from leading phasing correction parameters reference sample spot.

11. The method according to claim 10, wherein the phasing correction parameter further comprises at least one of lagging phasing correction parameters and leading phasing correction parameters, and the phasing correction parameter is obtained by training the following formula with a signal intensity value of each base channel from the plurality of second reference sample spots:

$$yi_{(B1,M)} = \beta 01 + B4 * Xi_{(B1,M-1)},$$

or

$$yi_{(B1,M)} = \beta 02 + B5 * Xi_{(B1,M+1)}$$

where:

B1 represents the given base channel, M represents the serial number of the given cycle, M+1 represents the number of the subsequent cycle of the given cycle, M-1 represents the number of the previous cycle of the given cycle,
$\beta 01$ and B4 represent lagging phasing correction parameters for the given base channel, and
$\beta 02$ and B5 represent leading phasing correction parameters for the given base channel; and
wherein the formula is trained with a regression model.

12. The method according to claim 1, further comprising inputting the corrected base-calling result as an input feature into a machine learning model to output a base combination of synchronous sequencing; wherein the machine learning model is trained in a supervised manner using a reference sequence with a predetermined sequence as a training set; wherein:

the reference sequence is subjected to synchronous sequencing, and a base-calling result obtained from a raw image of synchronous sequencing is corrected to generate a corrected base-calling result of each cycle as an input feature,
the base combination in the reference sequence corresponding to the base-calling result of each cycle is used as a label; and
the machine learning model is at least one of Bayesian, SVM, KNN, Random Forest, XGBoost, and Neural Network.

13. A method for correcting a base-calling result of synchronous sequencing, comprising:

acquiring a raw image set of the synchronous sequencing, wherein in the synchronous sequencing, at least one composite template sample spot is provided, at least one sequencing template is provided in the composite template sample spot, with a plurality of sequencing reaction cycles being performed on the at least one sequencing template, and an image set generated in each of the plurality of sequencing reaction cycles constitutes the raw image set;
acquiring a base-calling result of the synchronous sequencing based on the raw image set, wherein the base-calling result comprises a signal intensity value of each base channel in each of the plurality of sequencing reaction cycles; and
correcting the signal intensity value of each base channel based on a predetermined correction parameter to obtain a corrected base-calling result, wherein the correction parameter comprises at least one of a crosstalk correction parameter and a phasing correction parameter for each base channel.

**14.** The method according to claim 13, wherein the correction parameter is determined by:

identifying a plurality of high-confidence composite sample spots among the at least one composite template sample spot based on the base-calling result;

identifying, for a given base channel, a plurality of first reference sample spots and a plurality of second reference sample spots among the plurality of high-confidence composite sample spots, wherein the plurality of first reference sample spots are selected from crosstalk correction parameter reference sample spots and the plurality of second reference sample spots are selected from phasing correction parameter reference sample spots; and

identifying, for the given base channel, the crosstalk correction parameter of the given base channel based on a base-calling result of the plurality of first reference sample spots and the phasing correction parameter of the given base channel based on a base-calling result of the plurality of second reference sample spots,

wherein the plurality of high-confidence composite sample spots comprises a composite sample spot where the base-calling result indicates only one type of base in a given sequencing reaction cycle.

**15.** The method according to claim 14, wherein:

for the given base channel, the plurality of first reference sample spots comprise a composite sample spot satisfying the following condition:

in the given sequencing reaction cycle, the base-calling result of the composite sample spot indicates only one type of base that is different from the given base;

for the given base channel, the plurality of second reference sample spots are composite sample spots that satisfy the following conditions:

(A) in the given sequencing reaction cycle, the base-calling result of the composite sample spot indicates only one type of base that is different from the given base; and

(B) in at least one of the previous or subsequent cycles of the given sequencing reaction cycle, the base-calling result of the composite sample spot indicates only one type of the given base;

wherein in condition (B), if in the previous cycle of the given sequencing reaction cycle, the base-calling result of the composite sample spot indicates only one type of the given base, the second reference sample spot is identified as a third reference sample spot, wherein the third reference sample spot is selected from lagging phasing correction parameters reference sample spot; or

in condition (B), if in the subsequent cycle of the given sequencing reaction cycle, the base-calling result of the composite sample spot indicates only one type of the given base, the second reference sample spot is identified as a fourth reference sample spot, wherein the fourth reference sample spot is selected from leading phasing correction parameters reference sample spot.

**16.** The method according to claim 14, wherein:

the crosstalk correction parameter is obtained by training the following formula with a signal intensity value of each base channel from the plurality of first reference sample spots:

$$yi_{(B1,N)} = \beta0 + \beta1 * Xi_{(B2,N)} + \beta2 * Xi_{(B3,N)} + \beta3 * Xi_{(B4,N)} + \in$$

where:

B1, B2, B3, and B4 represent one of base A channel, base T channel, base G channel, and base C channel, respectively, with B1 representing the given base channel,

N represents the serial number of the given cycle,

$yi_{(B1,N)}$ represents the signal intensity value of the given base channel in the given cycle N,

$Xi_{(B2,N)}$, $Xi_{(B3,N)}$, and $Xi_{(B4,N)}$ represent signal intensity values of given base channels B2, B3, and B4 in the given cycle N, respectively,

$\beta0$, $\beta1$, $\beta2$, and $\beta3$ represent crosstalk correction parameters for the given base channel; and

$\in$ represents an error parameter;

the phasing correction parameter further comprises at least one of lagging phasing correction parameters and leading phasing correction parameters, and the phasing correction parameter is obtained by training the following formula with a signal intensity value of each base channel from the plurality of second reference sample spots:

$$yi_{(B1,M)} = \beta 01 + B4 * Xi_{(B1,M-1)},$$

or

$$yi_{(B1,M)} = \beta 02 + B5 * Xi_{(B1,M+1)}$$

where:

B1 represents the given base channel, M represents the serial number of the given cycle, M+1 represents the number of the subsequent cycle of the given cycle, M-1 represents the number of the previous cycle of the given cycle,

$\beta 01$ and B4 represent lagging phasing correction parameters for the given base channel, and

$\beta 02$ and B5 represent leading phasing correction parameters for the given base channel; and

the formula is trained with a regression model.

17. A system of synchronous sequencing, comprising:

a sequencing chip, provided with at least one composite template sample spot, the composite template sample spot being provided with at least one sequencing template;

a detection device, configured to perform a plurality of synchronous sequencing reaction cycles on a sequencing library, wherein an image set generated in each of the plurality of synchronous sequencing reaction cycles constitutes a raw image set of the synchronous sequencing; and

one or more processors, configured to execute:

(A) acquiring a base-calling result of the synchronous sequencing based on the raw image set of the synchronous sequencing, wherein the base-calling result comprises a signal intensity value of each base channel in each of the plurality of synchronous sequencing reaction cycles,

(B) correcting the signal intensity value of each base channel based on a predetermined correction parameter to obtain a corrected base-calling result, wherein the correction parameter comprises at least one of a crosstalk correction parameter and a phasing correction parameter for each base channel, and

(C) determining a base output result of the synchronous sequencing based on the corrected base-calling result.

18. The system according to claim 17, wherein the processor is further configured to execute a crosstalk correction parameter acquisition module, the crosstalk correction parameter acquisition module being configured to obtain the crosstalk correction parameter by training the following formula with the signal intensity value of each base channel from a plurality of first reference sample spots:

$$yi_{(B1,N)} = \beta 0 + \beta 1 * Xi_{(B2,N)} + \beta 2 * Xi_{(B3,N)} + \beta 3 * Xi_{(B4,N)} + \in$$

where:

B1, B2, B3, and B4 represent one of base A channel, base T channel, base G channel, and base C channel, respectively, with B1 representing the given base channel,

N represents the serial number of the given cycle,

$yi_{(B1,N)}$ represents the signal intensity value of the given base channel in the given cycle N,

$Xi_{(B2,N)}$, $Xi_{(B3,N)}$, and $Xi_{(B4,N)}$ represent signal intensity values of given base channels B2, B3, and B4 in the given cycle N, respectively,

$\beta 0$, $\beta 1$, $\beta 2$, and $\beta 3$ represent crosstalk correction parameters for the given base channel; and

$\in$ represents an error parameter; and

the plurality of first reference sample spots are selected from crosstalk correction parameter reference sample spots, and the plurality of first reference sample spots comprise a composite sample spot satisfying the following condition:

in the given sequencing reaction cycle, the base-calling result of the composite sample spot indicates only one type of base that is different from the given base.

19. The system according to claim 17, wherein the processor is further configured to execute a phasing correction parameter acquisition module, the phasing correction parameter acquisition module being configured to obtain the phasing correction parameter by training the following formula with the signal intensity value of each base channel from a plurality of second reference sample spots:

$$yi_{(B1,M)} = \beta01 + B4 * Xi_{(B1,M-1)},$$

or

$$yi_{(B1,M)} = \beta02 + B5 * Xi_{(B1,M+1)}$$

where:

B1 represents the given base channel, M represents the serial number of the given cycle, M+1 represents the number of the subsequent cycle of the given cycle, M-1 represents the number of the previous cycle of the given cycle,
$\beta01$ and B4 represent lagging phasing correction parameters for the given base channel, and
$\beta02$ and B5 represent leading phasing correction parameters for the given base channel; and
the plurality of second reference sample spots are composite sample spots that satisfy the following conditions:

(A) in the given sequencing reaction cycle, the base-calling result of the composite sample spot indicates only one type of base that is different from the given base; and
(B) in at least one of the previous or subsequent cycles of the given sequencing reaction cycle, the base-calling result of the composite sample spot indicates only one type of the given base.

20. An electronic device, comprising:

a memory, and
a processor;
wherein the memory stores a program that is executable by the processor, and the program, when executed by the processor, implements the method for correcting a base-calling result of synchronous sequencing according to any one of claims 13 to 16.

21. A computer-readable storage medium, storing one or more programs that are executable by one or more processors to implement the method for correcting a base-calling result of synchronous sequencing according to any one of claims 13 to 16.

S100

Constructing a sequencing library

S200

Loading

S300

Synchronous sequencing

S400

Acquiring base-calling result
of synchronous sequencing

S500

Correcting

S600

Determining base output result

FIG. 1

FIG. 2

FIG. 3

FIG. 4

Performing preliminary base-calling using synchronous data from standard *E. coli* libraries, and determining signal intensity and corresponding labels based on alignment results

Categorizing data based on maximum signal into four groups A, C, G, and T for separate modeling

Feature engineering for creating derived variables

Partitioning data into training and testing sets

Model training (XGBoost, SVM, Bayesian)

Applying models to raw data to obtain final base-calling results using individual models or through model ensemble methods

FIG. 5

Raw signal extraction of RInt

Selecting spots to calculate phasing and crosstalk coefficients

Noise removal to obtain Mint

Data normalization and signal stretching

Categorizing data based on the base with the maximum fluorescence intensity into four groups NA, NC, NG, and NT

whether the current cycle is the first sequencing cycle

Yes

No

Using models trained on intensities of 8 channels from the current cycle and the previous cycle

Using models trained on intensities of 4 channels from the current cycle

Base determination

Assigning base combinations to corresponding forward and reverse strands

FIG. 6

FIG. 7

FIG. 8

# EP 4 636 772 A1

## INTERNATIONAL SEARCH REPORT

| | International application No. |
|---|---|
| | **PCT/CN2022/138467** |

### A. CLASSIFICATION OF SUBJECT MATTER

G16B30/00(2019.01)i; C12Q1/68(2018.01)i

According to International Patent Classification (IPC) or to both national classification and IPC

### B. FIELDS SEARCHED

Minimum documentation searched (classification system followed by classification symbols)

G16B C12Q

Documentation searched other than minimum documentation to the extent that such documents are included in the fields searched

Electronic data base consulted during the international search (name of data base and, where practicable, search terms used)

CNTXT, ENTXTC, OETXT, DWPI, SIPOABS, CNABS, CNKI, PUBMED, web of knowledge: 同步测序, 双末端测序, 文库, 芯片, 图像, 串扰, 相移, 校正, 矫正, 学习模型, 碱基识别, basecalling, duplex sequencing, phasing, crosstalk, corrcection, rectification, chip, library

### C. DOCUMENTS CONSIDERED TO BE RELEVANT

| Category* | Citation of document, with indication, where appropriate, of the relevant passages | Relevant to claim No. |
|---|---|---|
| Y | CN 113012757 A (GENEMIND BIOSCIENCES CO., LTD.) 22 June 2021 (2021-06-22) abstract, and claims 1-10, and description, paragraphs 45 and 49-55 | 1-4, 12-13, 17, 20-21 |
| Y | CN 105392893 A (BGI SHENZHEN CO., LTD.) 09 March 2016 (2016-03-09) abstract, and claims 1-2, and description, page 2, drawings | 1-4, 12-13, 17, 20-21 |
| Y | CN 115035952 A (SHENZHEN SAILU MEDICAL TECHNOLOGY CO., LTD.) 09 September 2022 (2022-09-09) abstract, and claims 1-10, and description, paragraph 152 | 1-4, 12-13, 17, 20-21 |
| A | US 2010160172 A1 (COLD SPRING HARBOR LABORATORY) 24 June 2010 (2010-06-24) abstract, and claims 1-39 | 1-21 |
| A | US 2018195953 A1 (ILLUMINA, INC.) 12 July 2018 (2018-07-12) abstract, and claims 1-35 | 1-21 |
| A | SINGH, R. R. et al. "Next-Generation Sequencing in High-Sensitive Detection of Mutations in Tumors Challenges, Advances, and Applications" *The Journal of Molecular Diagnostics*, Vol. 22, No. 8, 31 August 2020 (2020-08-31), abstract, and pages 998-1000 | 1-21 |

☐ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **16 August 2023** | **22 August 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

| INTERNATIONAL SEARCH REPORT | International application No. |
|---|---|
| | **PCT/CN2022/138467** |

**Box No. I**      **Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

| Patent document cited in search report | | | Publication date (day/month/year) | Patent family member(s) | | | Publication date (day/month/year) |
|---|---|---|---|---|---|---|---|
| CN | 113012757 | A | 22 June 2021 | None | | | |
| CN | 105392893 | A | 09 March 2016 | WO | 2013053182 | A1 | 18 April 2013 |
| | | | | HK | 1215812 | A1 | 15 September 2016 |
| | | | | TW | 201315813 | A | 16 April 2013 |
| | | | | WO | 2013053183 | A1 | 18 April 2013 |
| | | | | WO | 2013053180 | A1 | 18 April 2013 |
| | | | | HK | 1193845 | A1 | 03 October 2014 |
| | | | | US | 2018371539 | A1 | 27 December 2018 |
| | | | | US | 2014249038 | A1 | 04 September 2014 |
| | | | | WO | 2013053207 | A1 | 18 April 2013 |
| CN | 115035952 | A | 09 September 2022 | None | | | |
| US | 2010160172 | A1 | 24 June 2010 | US | 8407012 | B2 | 26 March 2013 |
| US | 2018195953 | A1 | 12 July 2018 | PT | 3566158 | T | 23 June 2022 |
| | | | | US | 2021389236 | A1 | 16 December 2021 |
| | | | | BR | 112019013886 | A2 | 03 March 2020 |
| | | | | CA | 3049142 | A1 | 12 July 2018 |
| | | | | IL | 299500 | A | 01 February 2023 |
| | | | | RU | 2019122320 | A | 08 February 2021 |
| | | | | RU | 2019122320 | A3 | 27 May 2021 |
| | | | | RU | 2765996 | C2 | 07 February 2022 |
| | | | | IL | 291636 | A | 01 May 2022 |
| | | | | IL | 291636 | B2 | 01 June 2023 |
| | | | | KR | 20190104336 | A | 09 September 2019 |
| | | | | KR | 102385560 | B1 | 11 April 2022 |
| | | | | DK | 3566158 | T3 | 18 July 2022 |
| | | | | PL | 3566158 | T3 | 08 August 2022 |
| | | | | JP | 2020506677 | A | 05 March 2020 |
| | | | | JP | 7110207 | B2 | 01 August 2022 |
| | | | | ZA | 201904381 | B | 30 March 2022 |
| | | | | EP | 3566158 | A1 | 13 November 2019 |
| | | | | EP | 3566158 | B1 | 20 April 2022 |
| | | | | ES | 2917403 | T3 | 08 July 2022 |
| | | | | KR | 20230056053 | A | 26 April 2023 |
| | | | | KR | 20220047895 | A | 19 April 2022 |
| | | | | KR | 102521547 | B1 | 14 April 2023 |
| | | | | IL | 267799 | A | 26 September 2019 |
| | | | | IL | 267799 | B | 01 April 2022 |
| | | | | JP | 2022132542 | A | 08 September 2022 |
| | | | | HUE | 058858 | T2 | 28 September 2022 |
| | | | | LT | 3566158 | T | 27 June 2022 |
| | | | | MX | 2019008055 | A | 05 November 2019 |
| | | | | WO | 2018129314 | A1 | 12 July 2018 |
| | | | | AU | 2018205218 | A1 | 18 July 2019 |
| | | | | US | 11150179 | B2 | 19 October 2021 |

Form PCT/ISA/210 (patent family annex) (July 2022)